# EUROPEAN PATENT SPECIFICATION

(11) **EP 3 801 569 B1**
(45) Date of publication and mention of the grant of the patent: **04.11.2026**
(21) Application number: 19812475.2
(22) Date of filing: 28.05.2019
(51) Int. Cl.: A61M 1/02, A01N 1/147, A61K 35/12, A61K 35/28, A61K 35/36

(54) **METHODS AND SYSTEMS FOR CRYOPRESERVATION AND RESUSPENSION OF BODY FLUIDS**
VERFAHREN UND SYSTEME ZUR KRYOKONSERVIERUNG UND RESUSPENDIERUNG VON KÖRPERFLÜSSIGKEITEN
PROCÉDÉS ET SYSTÈMES DE CRYOCONSERVATION ET DE REMISE EN SUSPENSION DE LIQUIDES ORGANIQUES

(30) Priority: 31.05.2018 US 201862678765 P
(43) Date of publication of application: 14.04.2021
(73) Proprietor: Vitalant, Scottsdale AZ 85257 (US)
(72) Inventor: DUMONT, Larry J., Denver, Colorado 80230 (US)
(74) Representative: Kilburn & Strode LLP
(86) International application number: PCT/US2019/034136
(87) International publication number: WO 2019/231890

(56) References cited:
- RU-U1- 167 874
- US-A- 5 858 642
- US-A- 5 879 318
- US-A1- 2003 031 998
- US-A1- 2004 097 862
- US-A1- 2004 097 862
- US-A1- 2012 219 633
- JOHNSON ET AL.: "PAS-G supports platelet reconstitution after cryopreservation in the absence of plasma", TRANSFUSION, 1 October 2013 (2013-10-01), pages 2268 - 2277, XP055659577, ISSN: 0041-1132, DOI: 10.1111/trf.12084
- "Cryopreservation Biotechnology in Biomedical and Biological Sciences", 12 December 2018, ISBN: 978-1-78984-681-2, article BOHONEK, MILOS: "Cryopreservation of Platelets: Advances and Current Practice", pages: 47 - 70, XP055659582, DOI: 10.5772/intechopen.81906

## Description

### CROSS-REFERENCE TO RELATED PATENT APPLICATION

This patent application claims the benefit under 35 U.S.C. § 119(e) of U.S. Patent Application Serial No. 62/678,765, entitled "METHODS AND SYSTEMS FOR CRYOPRESERVATION AND RESUSPENSION OF BODY FLUIDS," filed on May 31, 2018.

### FIELD

The present disclosure is directed to a cryopreservation and resuspension system for body fluids and methods of use thereof. Prior art examples are disclosed e.g. in RU167874 U1, US2012/0219633 A1 or US2004/0097862 A1.

### BACKGROUND

Blood products and other body fluids or biologicals may be cryopreserved for long term storage. The preparation of the product for freezing may be in a manner to prevent contamination of the product, for example with aseptic handling and filling operations or through the use of sterile connection devices. After the product is thawed through any variety of means, e.g. water bath, the product must be prepared for administration/transfusion which may require dilution, washing, centrifugation, filtration, resuspension or similar steps that require accessing the product storage container. These processes may breach the closed system of the storage container, and therefore subject the product to potential contamination.

This presents an increased risk to the product and the patient, and limits the time interval between preparation and administration/transfusion to 4-6 hours depending on the regulatory jurisdiction. These operations could be performed in a validated aseptic handling environment (i.e., clean room). However, many locations of final use do not have the validated facilities and trained personnel to aseptically process in a controlled environment (e.g., some hospitals, rural health care settings, home settings, remote emergency care, or austere environments such as military deployment zone). Functionally closed operations may be facilitated by the use of sterile tube welding. But, some freezing storage systems are not conducive to a device design/material selection that will permit compatibility to approved sterile connection devices.

For example, conventional resuspension operations necessitate breaching the storage container may result in a 4-6 hour expiry time for the thawed cells, such as cryopreserved platelets (CPP). This can result in waste of valuable product if the product is not administered to the patient within that time window.

Accordingly, there remains a need in developing a system and method for freezing, resuspending body fluids in a closed system such that the contents of the system remain sterile through thawing, resuspension, and storage until used by a patient.

### BRIEF SUMMARY

The disclosure provides for container systems and methods of use thereof. The invention is defined by the appended claims.

Additional variations and features are set forth in part in the description that follows, and will become apparent to those skilled in the art upon examination of the specification or may be learned by the practice of the disclosed subject matter. A further understanding of the nature and advantages of the disclosure may be realized by reference to the remaining portions of the specification and the drawings, which forms a part of this disclosure.

### BRIEF DESCRIPTION OF THE DRAWINGS

The description will be more fully understood with reference to the following figures and data graphs, which are presented as variations of the disclosure and should not be construed as a complete recitation of the scope of the disclosure, wherein:
**FIG. 1** is an illustration of the container system in one variation.
**FIG. 2** is an illustration of the container system in one variation.
**FIG. 3** is a block diagram for a method of preparing a frozen body fluid in the container system of FIG. 1.
**FIG. 4** is a block diagram for a method of preparing a frozen body fluid in the container system of FIG. 2.

### DETAILED DESCRIPTION

The disclosure may be understood by reference to the following detailed description, taken in conjunction with the drawings as described below. It is noted that, for purposes of illustrative clarity, certain elements in various drawings may not be drawn to scale.

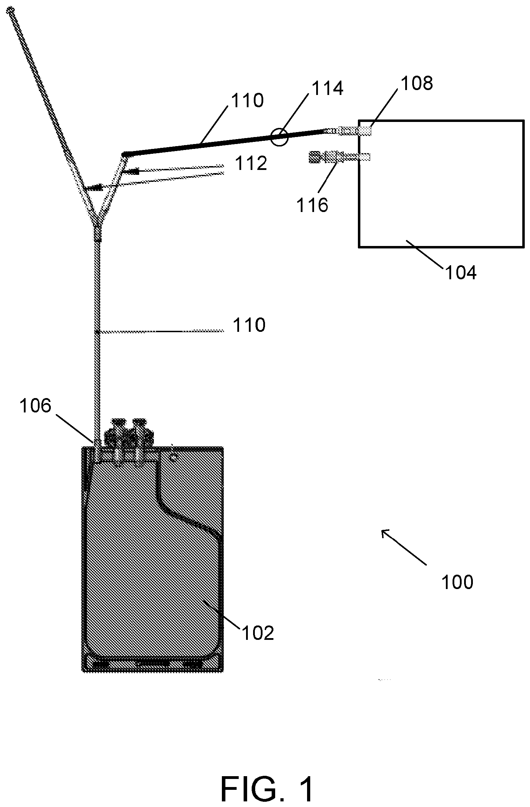

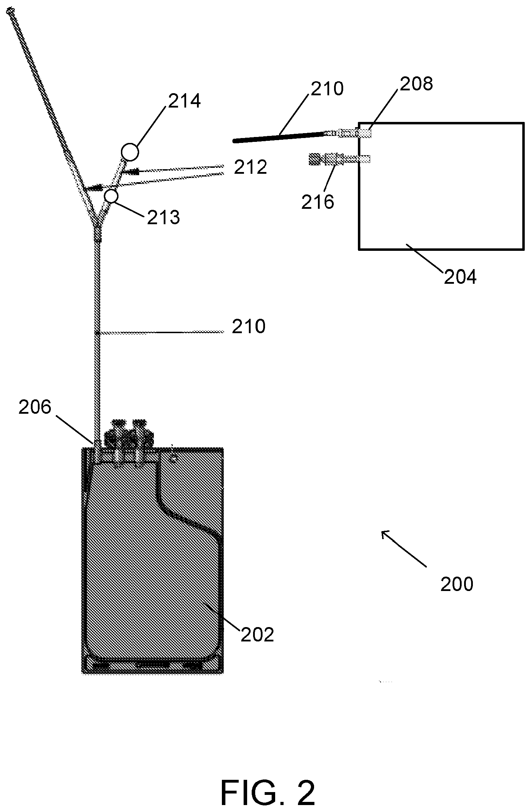

Disclosed herein are container systems and methods for the cryopreservation and resuspension of a body fluid. In one variation, as seen in **FIG. 1****,** the container system may include a cryopreservation container, a resuspension container, a connection tube sterilely connecting the cryopreservation container and the resuspension container, and at least one shut-off element actively associated with the connection tube. The container system may be frozen together for up to 2 to 10 years depending on the body fluid. In another variation, as seen in **FIG. 2****,** the container system includes a cryopreservation container and a sterile filter sterilely and fluidly connected to the cryopreservation container. This container system may further include a resuspension container configured to connect to the sterile filter after thawing.

The systems do not require a sterile connection/tube welding post thawing and do not require a validated, controlled environment for aseptic filling or sterilization operations. With the use of the systems with appropriate solutions, the final thawed and prepared body fluid product can be maintained for an extended period of time under appropriate conditions to aid in the treatment logistics. The system may prevent loss of body fluid product because the thawed and resuspended body fluid product may be sterile and stable for use more than 4-6 hours post preparation. For example, platelets resuspended using the container systems described herein may have a shelf life of greater than 4 hours up to at least 14 days. Other resuspended body fluids may have a shelf life of at least 3 months.

The thawed and prepared body fluid product can also be transported to the site of treatment under appropriate conditions. Non-limiting examples of treatment sites include transfusion services at hospitals, first responders in ambulances or helicopters, medical transportation and evacuation carriers, first responders on ground operations such as special forces. The extended shelf life may be important for civilian applications and military application. By extending the expiry time following thawing, the logistical preparation challenges are greatly relieved, reducing wastage, and permitting movement of the product to the site of care which could be remote from the site of storage and preparation. The thawed body fluid may be used to treat a patient in need of the body fluid. In one example, thawed CPP within the container system may be used treatment of acute hemorrhage in patients with a platelet deficiency or a platelet dysfunction.

### Container System with Two Sterile Connected Containers

Provided herein is a container system for the cryopreservation and resuspension of a body fluid. As seen in **FIG. 1****,** the container system **100** includes a cryopreservation container **102,** a resuspension container **104,** a connection tube **110** sterilely connecting the cryopreservation container and the resuspension container, and at least one shut-off element **114** actively associated with the connection tube.

The system includes a resuspension container pre-attached to a cryopreservation or freezing container prior to freezing such that a closed container system is available to thaw and prepare a body fluid product for administration/transfusion. The container system is prepared dry and sterilized by any manner of validated methods including but not limited to ethylene oxide, electron beam, gamma irradiation to form a sterile assembly.

The cryopreservation container **102** may be any container capable of holding a frozen body fluid. The cryopreservation container may be pliable or rigid. Non-limiting examples of materials that may compose the cryopreservation container include ethyl vinyl acetate (EVA), polyvinylchloride (PVC), polytetrafluoroethylene (PTFE), polyolefin polymers, such as polyethylene, and combinations thereof. In one variation, the cryopreservation container may be a cryopreservation bag. The cryopreservation bag is compatible with a cryopreservation fluid. For example, the cryopreservation bag is dimethyl sulfoxide (DMSO) compatible. In a variation, the cryopreservation container may be EVA freezing bag.

The cryopreservation container **102** may have one or more sterile connectors fluidly connected to the container. In a variation, the cryopreservation container includes at least 1 sterile connector. In a variation, the cryopreservation container includes at least 2 sterile connectors. In a variation, the cryopreservation container includes at least 3 sterile connectors. The sterile connectors may provide for sterilely connecting other containers to the cryopreservation container such that the contents of one container may be sterilely transferred to another container. The sterile connectors permit sterile welding of tubing for fluid transfers and/or connections for aseptic filling of fluids. In a variation, the sterile connectors are DMSO compatible. The cryopreservation container may have a volume sufficient to hold a desired amount of body fluid for transfusion. In a variation, the cryopreservation container may have a volume of at least 1 mL. In a variation, the cryopreservation container may have a volume of at least 5 mL. In a variation, the cryopreservation container may have a volume of at least 10 mL. In a variation, the cryopreservation container may have a volume of at least 25 mL. In a variation, the cryopreservation container may have a volume of at least 100 mL. In a variation, the cryopreservation container may have a volume of at least 200 mL. In a variation, the cryopreservation container may have a volume of at least 500 mL. In a variation, the cryopreservation container may have a volume of at least 800 mL. In a variation, the cryopreservation container may have a volume of at least 1000 mL.

The cryopreservation container contains a body fluid in a cryopreservation liquid. In a variation, the body fluid is added to the cryopreservation container though sterile tubing welds or other aseptic methods of transferring fluids. Non-limiting examples of body fluids that may be contained within the cryopreservation container include platelets, whole blood, red blood cells, plasma, cord blood, stem cells, mesenchymal stromal/stem cells, other biologicals, and/or combinations thereof. The body fluid may be human or animal body fluid for use in humans or animals. In one variation, the body fluid is platelets in plasma that have been collected from healthy volunteer allogeneic donors. The body fluid may be sterilized, if required. In a variation, collected platelets may be irradiated with 25Gy.

In a variation, the body fluid is combined with a cryopreservation fluid in the cryopreservation container. The cryopreservation fluid may be added to the body fluid aseptically in a clean room (e.g., ISO 5 classified) or using sterile welding of a cryopreservation fluid container to the cryopreservation container containing the body fluid. Examples of the cryopreservation fluid include DMSO, glycerol, or any fluid capable of preserving a frozen body fluid. In one variation, the cryopreservation fluid is 27% DMSO. In one variation, the body fluid mixture is DMSO cryopreserved platelets (CPP).

The cryopreservation fluid may be added such that it is 3% to 10% by volume of the total mixture. In a variation, the cryopreservation fluid may be at least 3% by volume of the mixture with the body fluid. In a variation, the cryopreservation fluid may be at least 6% by volume of the mixture with the body fluid. In a variation, the cryopreservation fluid may be at least 8% by volume of the mixture with the body fluid. In a variation, the cryopreservation fluid may be at least 10% by volume of the mixture with the body fluid. In another variation, the cryopreservation fluid may be less than or equal to 3% by volume of the mixture with the body fluid. In another variation, the cryopreservation fluid may be less than or equal to 6% by volume of the mixture with the body fluid. In another variation, the cryopreservation fluid may be less than or equal to 8% by volume of the mixture with the body fluid. In another variation, the cryopreservation fluid may be less than or equal to 10% by volume of the mixture with the body fluid.

After the cryopreservation fluid is added to the body fluid, the mixture may be concentrated within the cryopreservation bag, such as by centrifugation. The supernatant may then be removed from the cryopreservation bag so that the volume of the body fluid mixture in the cryopreservation bag is less than the starting volume of the body fluid and cryopreservation fluid. In a variation, the volume of concentrated body fluid mixture to be frozen is at least 1 mL. In a variation, the volume of concentrated body fluid mixture to be frozen is at least 5 mL. In a variation, the volume of concentrated body fluid mixture to be frozen is at least 10 mL. In a variation, the volume of concentrated body fluid mixture to be frozen is at least 15 mL. In a variation, the volume of concentrated body fluid mixture to be frozen is at least 20 mL. In a variation, the volume of concentrated body fluid mixture to be frozen is at least 25 mL. In a variation, the volume of concentrated body fluid mixture to be frozen is at least 35 mL. In a variation, the volume of concentrated body fluid mixture to be frozen is at least 50 mL. In a variation, the volume of concentrated body fluid mixture to be frozen is at least 100 mL. In other variations the volume of concentrated body fluid mixture to be frozen is less than or equal to 20 mL. In other variations the volume of concentrated body fluid mixture to be frozen is less than or equal to 25 mL. In other variations the volume of concentrated body fluid mixture to be frozen is less than or equal to 35 mL. In other variations the volume of concentrated body fluid mixture to be frozen is less than or equal to 50 mL. In other variations the volume of concentrated body fluid mixture to be frozen is or less than or equal to 100 mL. For example, platelets may be concentrated to 20 mL to 35 mL by centrifugation in the cryopreservation container.

The container system **100** further includes a resuspension container **104** having a resuspension solution contained within the resuspension container. The resuspension container may be any container capable of being frozen. The resuspension container may be pliable or rigid. Non-limiting examples of materials that may compose the resuspension container include EVA, PVC, PTFE, polyolefin polymers, such as polyethylene, and combinations thereof. In one variation, the resuspension container may be a bag. The resuspension bag may be compatible with the cryopreservation fluid. For example, the resuspension bag is DMSO compatible. In a variation, the cryopreservation container may be an EVA freezing bag.

The resuspension container **104** may have one or more sterile connectors **108** fluidly connected to the container. In a variation, the resuspension container includes at least 1 sterile connector. In a variation, the resuspension container includes at least 2 sterile connectors. In a variation, the resuspension container includes at least 3 sterile connectors. The sterile connectors may provide for sterilely connecting the cryopreservation container to the resuspension container such that the contents of the resuspension container may be sterilely transferred to the cryopreservation container. In a variation, the resuspension container may include sterile connectors **116** which permit sterile welding of tubing for fluid transfers and/or connections for aseptic filling of fluids. In a variation, the sterile connectors are DMSO compatible. Non-limiting examples of sterile connectors include N/F valves and non-venting valves. The resuspension container may have a volume sufficient to hold a desired amount of resuspension solution for transfusion. In a variation, the resuspension container may have a volume of less than or equal to 15 mL. In a variation, the resuspension container may have a volume of at least 15 mL. In a variation, the resuspension container may have a volume of at least 30 mL. In a variation, the resuspension container may have a volume of at least 50 mL. In a variation, the resuspension container may have a volume of at least 100 mL. In a variation, the resuspension container may have a volume of at least 500 mL. In a variation, the resuspension container may have a volume of at least 1000 mL.

The resuspension solution may be any biocompatible solution sufficient to be mixed with the body fluid for introduction into the body. In a variation, the resuspension solution is a validated, transfusable solution. Non-limiting examples of transfusable solutions include sterile NaCl, human plasma, and a platelet storage solution. In one variation, the resuspension solution is 0.9% NaCl. In some variations, the sterile resuspension solution is added to the resuspension container either with a sterile connection or through a sterile filling operation in a controlled environment. In other variations, the resuspension container is filled followed by a terminal sterilization by steam, gamma, ebeam, etc.

The container system **100** further includes a connection tube **110** sterilely connecting the cryopreservation container and the resuspension container. The connection tube is configured to withstand being clamped and frozen, e.g. at -80°C. In a variation, the connection tube may be DMSO compatible. The connection tube may include but is not limited to EVA, PVC, or combinations thereof. In one variation, the connection tube is a co-extruded PVC/EVA tube. The connection tube may be sterile welded to any sterile connectors or attachments.

The connection tube has a length sufficient to store the cryopreservation container and the resuspension container in the connected state. In a variation, the length of the connection tube is at least 5 cm. In a variation, the length of the connection tube is at least 10 cm. In a variation, the length of the connection tube is at least 15 cm. In a variation, the length of the connection tube is at least 20 cm. In a variation, the length of the connection tube is at least 25 cm. In a variation, the length of the connection tube is at least 30 cm. In a variation, the length of the connection tube is at least 40 cm. In a variation, the length of the connection tube is at least 50 cm. In other variations, the length of the connection tube is less than or equal to 5 cm. In other variations, the length of the connection tube is less than or equal to 10 cm. In other variations, the length of the connection tube is less than or equal to 15 cm. In other variations, the length of the connection tube is less than or equal to 20 cm. In other variations, the length of the connection tube is less than or equal to 25 cm. In other variations, the length of the connection tube is less than or equal to 30 cm. In other variations, the length of the connection tube is less than or equal to 40 cm. In other variations, the length of the connection tube is less than or equal to 50 cm. In one variation, the connection tube is 19 cm in length. In other variations, the connection tube may have multiple segments or be split with a Y-adapter **112.** For example, a first section of the connection tube may be connected to a sterile connector on the cryopreservation container at a first end and connected to a Y-adapter at a second end. A second section of connection tube may be connected to one branch of the Y-adapter at a first end and connected to a sterile connector on the resuspension container at a second end. The first and second sections of the connection tube may be made of different materials.

The container system **100** further includes at least one shut-off element **114** actively associated with the connection tube. The shut-off element is configured to remain in the closed position when frozen, e.g. at -80°C. In a variation, the shut-off element may be a clamp, valve (e.g., stop cock), breakable fitting (frangible), or other appropriate fitting that will allow sterile opening of the tubing between the containers for fluid transfer. In one example, the shut-off element is a roller clamp. The shut-off element is configured to separate the contents of the cryopreservation container and the resuspension container when in the off position and allow mixing of the contents in the open position. In a variation, the container system is frozen and the shut-off element is closed such that the body fluid in a cryopreservation liquid and resuspension solution cannot mix. In another variation, the frozen container system is thawed, the shut-off element is opened, and at least a portion of the resuspension solution is added to the cryopreservation container.

The container system may be frozen in the sterilely connected state until the body fluid is needed to be thawed and transfused. The sterilely connected state includes the cryopreservation container and the resuspension container connected with the connection tube. In some variations, the container system is frozen at a temperature of less than or equal to -15°C to at least -196°C. In a variation, the container system is frozen to a temperature of less than or equal to -15°C. In a variation, the container system is frozen to a temperature of less than or equal to -25°C. In a variation, the container system is frozen to a temperature of less than or equal to -50°C. In a variation, the container system is frozen to a temperature of less than or equal to -65°C. In a variation, the container system is frozen to a temperature of less than or equal to -70°C. In a variation, the container system is frozen to a temperature of less than or equal to -75°C. In a variation, the container system is frozen to a temperature of less than or equal to -80°C. In a variation, the container system is frozen to a temperature of less than or equal to -100°C. In a variation, the container system is frozen to a temperature of less than or equal to -150°C. In a variation, the container system is frozen to a temperature of less than or equal to -196°C. In one variation, the container system is frozen by placing it in a cardboard plasma freezing container, freezing it in a chest-type mechanical freezer set at -80°C, and holding it frozen at -65°C. The container system may be frozen for up to 2 to up to 10 years depending on the body fluid.

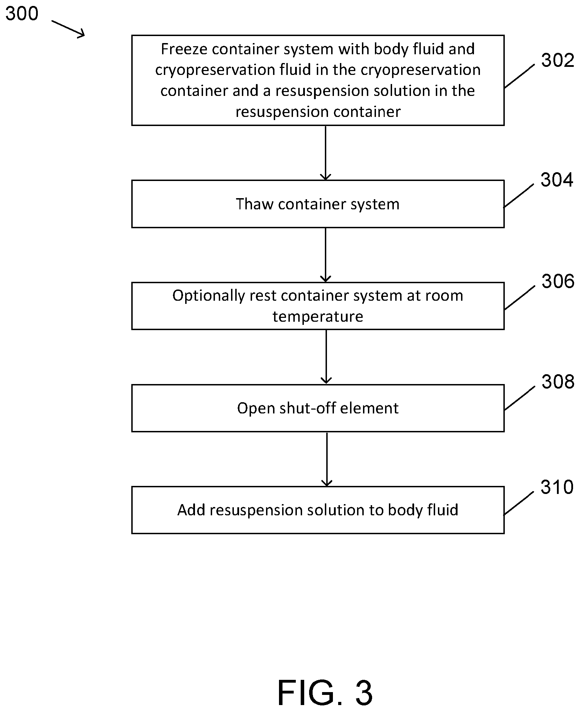

**FIG. 3** provides a method **300** of preparing a frozen body fluid for transfusion into a patient. The method may include freezing the container system at step **302,** thawing the contents of both the cryopreservation container and the resuspension container at step **304,** opening the shut-off element at step **308,** and mixing a portion of the resuspension solution into the body fluid at step **310.** Thawing can be with conductive or radiant heat with or without simultaneous mixing. Methods of thawing include, but are not limited to water baths or microwave heating. In an example, frozen CPP are prepared for transfusion by thawing in a 30-37°C water bath and resuspended in a resuspension solution. In a variation, the resuspension solution is sterile 0.9% NaCl for infusion or plasma. The resuspension solution is added at a validated time period following thawing, which may be immediately or after an extended rest of the system. The method may optionally include a rest period of up to 30 minutes at room temperature at step 306 after thawing and before adding the resuspension solution. The need for the rest period may be based on the selection of the resuspension solution.

After the contents of the containers are combined, the connection tubing is sealed with a radio frequency sealer, heat sealer, or clamp. The final product may be held under appropriate conditions and/or transported until the time of transfusion that will not be limited due to a breach of the closed system. It may be limited based on other biological and physiochemical features of the final product such as temperature, cell concentration in the bag, the final resuspension solution, gas permeability of the container and others.

In a variation, the thawed, resuspended body fluid has a shelf life of more than 4 hours. In a variation, the resuspended body fluid has a shelf life of at least 4 hours. In a variation, the resuspended body fluid has a shelf life of at least 12 hours. In a variation, the resuspended body fluid has a shelf life of at least 24 hours. In a variation, the resuspended body fluid has a shelf life of at least 2 days. In a variation, the resuspended body fluid has a shelf life of at least 5 days. In a variation, the resuspended body fluid has a shelf life of at least 2 weeks. In a variation, the resuspended body fluid has a shelf life of at least 1 month. In a variation, the resuspended body fluid has a shelf life of at least 2 months. In a variation, the resuspended body fluid has a shelf life of at least 3 months. In some variations, expiry of the resuspended body fluid may be a function of the resuspension solution type, volume of the resuspended body fluid, the content of the body fluid, etc.

### Container System with Integrated Sterile Filter

Provided herein is a sterile container system for the cryopreservation and resuspension of a body fluid with an integrated sterile filter. Referring to **FIG. 2****,** the container system **200** includes a cryopreservation container **202** and a sterile filter **214** sterilely and fluidly connected to the cryopreservation container. The cryopreservation container **202** may contain the body fluid in a cryopreservation liquid. In a variation, the sterile filter **214** is connected to the cryopreservation container **202** prior to freezing the cryopreservation container. In a variation, the container system **200** may further include a resuspension container **204** configured to connect to the sterile filter **214.** The resuspension container **204** may contain a resuspension solution. The resuspension container may or may not be frozen. The resuspension container may or may not be pre-attached or frozen with the cryopreservation container. In a variation, the connection from the resuspension container to the sterile filter may be with another device (solution administration set) or may be integrally attached to the resuspension container. For example, the resuspension container may connect to the sterile filter via a luer connection. In another variation, the sterile filter may be configured with a sterile dockable piece of tubing on the distal port from cryopreservation container allowing a sterile connection to the filter.

The system includes a sterile filter pre-attached to a cryopreservation or freezing container prior to freezing such that a closed container system is available to thaw and prepare a body fluid product for administration/transfusion. The container system is prepared dry and sterilized by any manner of validated methods including but not limited to ethylene oxide, electron beam, gamma irradiation to form a sterile assembly.

The cryopreservation container **202** may be any container capable of holding a frozen body fluid. The cryopreservation container may be pliable or rigid. Non-limiting examples of materials that may compose the cryopreservation container include EVA, PVC, PTFE, polyolefin polymers, such as polyethylene, and combinations thereof. In one variation, the cryopreservation container may be a cryopreservation bag. The cryopreservation bag is compatible with a cryopreservation fluid. For example, the cryopreservation bag is DMSO compatible. In a variation, the cryopreservation container may be an ethyl vinyl acetate (EVA) freezing bag.

The cryopreservation container **202** may have one or more sterile connectors **206** fluidly connected to the container. In a variation, the cryopreservation container includes at least 1 sterile connector. In a variation, the cryopreservation container includes at least 2 sterile connectors. In a variation, the cryopreservation container includes at least 3 sterile connectors. The sterile connectors **206** may provide for sterilely connecting other containers to the cryopreservation container such that the contents of one container may be sterilely transferred to another container. The sterile connectors permit sterile welding of tubing for fluid transfers and/or connections for aseptic filling of fluids. In a variation, the sterile connectors are DMSO compatible. The cryopreservation container may have a volume sufficient to hold a desired amount of body fluid for transfusion. In a variation, the cryopreservation container may have a volume of at least 1 mL. In a variation, the cryopreservation container may have a volume of at least 5 mL. In a variation, the cryopreservation container may have a volume of at least 10 mL. In a variation, the cryopreservation container may have a volume of at least 25 mL. In a variation, the cryopreservation container may have a volume of at least 200 mL. In a variation, the cryopreservation container may have a volume of at least 500 mL. In a variation, the cryopreservation container may have a volume of at least 800 mL. In a variation, the cryopreservation container may have a volume of at least 1000 mL.

The cryopreservation container contains a body fluid in a cryopreservation liquid. Non-limiting examples of body fluids that may be contained within the cryopreservation container include platelets, whole blood, red blood cells, plasma, cord blood, stem cells, mesenchymal stromal/stem cells, other biologicals, and combinations thereof. The body fluid may be human or animal body fluid for use in humans or animals. In one variation, the body fluid is platelets in plasma that have been collected from healthy volunteer allogeneic donors. In a variation, the collected platelets may be irradiated with 25Gy.

In a variation, the body fluid is combined with a cryopreservation fluid in the cryopreservation container. The cryopreservation fluid may be added to the body fluid aseptically in a clean room (e.g., ISO 5 classified) or using sterile welding of a cryopreservation fluid container to the cryopreservation container containing the body fluid. Examples of the cryopreservation fluid include DMSO, glycerol, or any fluid capable of preserving a frozen body fluid, such as a cell freezing medium. In one variation, the cryopreservation fluid is 27% DMSO. In one variation, the body fluid mixture is DMSO cryopreserved platelets (CPP). The cryopreservation fluid may be added such that it is 3% to 10% by volume of the total mixture. In a variation, the cryopreservation fluid may be at least 3% by volume of the mixture with the body fluid. In a variation, the cryopreservation fluid may be at least 6% by volume of the mixture with the body fluid. In a variation, the cryopreservation fluid may be at least 8% by volume of the mixture with the body fluid. In a variation, the cryopreservation fluid may be at least 10% by volume of the mixture with the body fluid. In another variation, the cryopreservation fluid may be less than or equal to 3% by volume of the mixture with the body fluid. In another variation, the cryopreservation fluid may be less than or equal to 6% by volume of the mixture with the body fluid. In another variation, the cryopreservation fluid may be less than or equal to 8% by volume of the mixture with the body fluid. In another variation, the cryopreservation fluid may be less than or equal to 10% by volume of the mixture with the body fluid.

After the cryopreservation fluid is added to the body fluid, the mixture may be concentrated within the cryopreservation bag, such as by centrifugation. The supernatant may then be removed from the cryopreservation bag so that the volume of the body fluid mixture in the cryopreservation bag is less than the starting volume of the body fluid and cryopreservation fluid. In a variation, the volume of concentrated body fluid mixture to be frozen is at least 1 mL. In a variation, the volume of concentrated body fluid mixture to be frozen is at least 5 mL. In a variation, the volume of concentrated body fluid mixture to be frozen is at least 10 mL. In a variation, the volume of concentrated body fluid mixture to be frozen is at least 15 mL. In a variation, the volume of concentrated body fluid mixture to be frozen is at least 20 mL. In a variation, the volume of concentrated body fluid mixture to be frozen is at least 25 mL. In a variation, the volume of concentrated body fluid mixture to be frozen is at least 35 mL. In a variation, the volume of concentrated body fluid mixture to be frozen is at least 50 mL. In a variation, the volume of concentrated body fluid mixture to be frozen is at least 100 mL. In other variations the volume of concentrated body fluid mixture to be frozen is less than or equal to 20 mL. In other variations the volume of concentrated body fluid mixture to be frozen is less than or equal to 25 mL. In other variations the volume of concentrated body fluid mixture to be frozen is less than or equal to 35 mL. In other variations the volume of concentrated body fluid mixture to be frozen is less than or equal to 50 mL. In other variations the volume of concentrated body fluid mixture to be frozen is or less than or equal to 100 mL. For example, platelets may be concentrated to 20 mL to 35 mL by centrifugation in the cryopreservation container.

The container system **200** further includes a sterile filter **214** sterilely connected to the cryopreservation container **202.** The sterile filter **214** is configured to sterilely filter any fluid, such as a resuspension solution that is added to the cryopreservation container. In an example, the sterile filter **214** has a pore size of less than or equal to 0.22 µm. In one variation, the sterile filter has ultrafiltration cutoff of 0.1 µm. The sterile filter **214** may be a membrane filter (non-woven, woven or other means of making holes in a membrane of less than 0.22 µm) or a semipermeable dialysis type membrane. In a variation, the sterile filter is integrated with the cryopreservation container. The sterile filter **214** may be connected to a sterile connector **206** on the cryopreservation container or connected to a connection tube **210** that is fluidly connected to the sterile connector. The sterile filter is integrated with the cryopreservation container such that the connection remains sterile throughout the freezing and thawing processes. The sterile filter is capable of being frozen down to at least -80°C or at least -196°C without impacting the functionality of the filter once thawed. In a variation, the sterile filter may have a Leur connection for connecting the resuspension container to the sterile filter.

In some variations, the connection tube **210** may have multiple segments or be split with a Y-adapter **212.** For example, a first section of the connection tube may be connected to a sterile connector on the cryopreservation container at a first end and connected to a Y-adapter at a second end. A second section of connection tube may be connected to one branch of the Y-adapter at a first end and connected to a sterile filter at a second end. The first and second sections of the connection tube may be made of different materials. In another variation, the sterile filter may be directly connected to a branch of the Y-adapter.

The container system **200** may further include at least one shut-off element **213** actively associated with the connection tube or Y-adapter. The shut-off element is configured to remain in the closed position when frozen, e.g. at -80°C. In a variation, the shut-off element may be a clamp, valve (e.g., stop cock), breakable fitting (frangible), or other appropriate fitting that will allow sterile opening of the tubing between the cryopreservation container and sterile filter for fluid transfer. In one example, the shut-off element is a roller clamp. The shut-off element is configured to prevent the contents of the cryopreservation container from entering the sterile filter when in the off position and allow passage of the resuspension solution in the open position. In a variation, the container system is frozen and the shut-off element is closed such that the body fluid in a cryopreservation liquid cannot reach the sterile filter. In another variation, the frozen container system is thawed, the shut-off element is opened, and at least a portion of the resuspension solution is added to the cryopreservation container through the sterile filter.

The container system **200** further includes a resuspension container **204** having a resuspension solution contained within the resuspension container. The resuspension container may be any container capable of being frozen. The resuspension container may be pliable or rigid. Non-limiting examples of materials that may compose the resuspension container include EVA, PVC, PTFE, polyolefin polymers, such as polyethylene, and combinations thereof. In one variation, the resuspension container may be a bag. In another variation, the resuspension container is a syringe. The resuspension container may be compatible with the cryopreservation fluid. For example, the resuspension container is DMSO compatible. In a variation, the cryopreservation container may be an EVA freezing bag.

The resuspension solution may be any biocompatible solution sufficient to be mixed with the body fluid for introduction into the body. In a variation, the resuspension solution is a verified, transfusable solution. Non-limiting examples of transfusable solutions include sterile NaCl, human plasma, and a platelet storage solution. In one variation, the resuspension solution is 0.9% NaCl. In some variations, the sterile resuspension solution is added to the resuspension container either with a sterile connection or through a sterile filling operation in a controlled environment. In other variations, the resuspension container is filled followed by a terminal sterilization by steam, gamma, ebeam, etc.

The resuspension container **204** may have one or more sterile connectors **208** fluidly connected to the container. In a variation, the resuspension container includes at least 1 sterile connector. In a variation, the resuspension container includes at least 2 sterile connectors. In a variation, the resuspension container includes at least 3 sterile connectors. The sterile connectors may provide for sterilely connecting the resuspension container to the sterile filter **214** such that the contents of the resuspension container may be sterilely transferred to the cryopreservation container. In a variation, the resuspension container may include sterile connectors **216** which permit sterile welding of tubing for fluid transfers and/or connections for aseptic filling of fluids. In a variation, the sterile connectors are DMSO compatible. Non-limiting examples of sterile connectors include N/F valves and non-venting valves. The resuspension container may have a volume sufficient to hold a desired amount of resuspension solution for transfusion. In a variation, the resuspension container may have a volume of less than or equal to 15 mL. In a variation, the resuspension container may have a volume of at least 15 mL. In a variation, the resuspension container may have a volume of at least 30 mL. In a variation, the resuspension container may have a volume of at least 50 mL. In a variation, the resuspension container may have a volume of at least 100 mL.

In a variation, the sterile connector **208** of the resuspension container **204** may be connected to a connection tube, which is then connected to the sterile filter 214. In a variation, the connection tube may be DMSO compatible. The connection tube may include but is not limited to EVA, PVC, or combinations thereof. In one variation, the connection tube is a co-extruded PVC/EVA tube. The connection tube may be sterile welded to any sterile connectors or attachments.

In a variation, the length of the connection tube is at least 5 cm. In a variation, the length of the connection tube is at least 10 cm. In a variation, the length of the connection tube is at least 15 cm. In a variation, the length of the connection tube is at least 20 cm. In a variation, the length of the connection tube is at least 25 cm. In a variation, the length of the connection tube is at least 30 cm. In a variation, the length of the connection tube is at least 40 cm. In a variation, the length of the connection tube is at least 50 cm. In other variations, the length of the connection tube is less than or equal to 5 cm. In other variations, the length of the connection tube is less than or equal to 10 cm. In other variations, the length of the connection tube is less than or equal to 15 cm. In other variations, the length of the connection tube is less than or equal to 20 cm. In other variations, the length of the connection tube is less than or equal to 25 cm. In other variations, the length of the connection tube is less than or equal to 30 cm. In other variations, the length of the connection tube is less than or equal to 40 cm. In other variations, the length of the connection tube is less than or equal to 50 cm. In one variation, the connection tube is 19 cm in length.

The container system may be frozen in the sterilely connected state until the body fluid is needed to be thawed and transfused. The sterilely connected state includes the cryopreservation container with the integrated sterile filter. In some variations, the container system is frozen at a temperature of less than or equal to -15°C to at least -196°C. In a variation, the container system is frozen to a temperature of less than or equal to -15°C. In a variation, the container system is frozen to a temperature of less than or equal to -25°C. In a variation, the container system is frozen to a temperature of less than or equal to -50°C. In a variation, the container system is frozen to a temperature of less than or equal to -65°C. In a variation, the container system is frozen to a temperature of less than or equal to -70°C. In a variation, the container system is frozen to a temperature of less than or equal to -75°C. In a variation, the container system is frozen to a temperature of less than or equal to -80°C. In a variation, the container system is frozen to a temperature of less than or equal to -100°C. In a variation, the container system is frozen to a temperature of less than or equal to -150°C. In a variation, the container system is frozen to a temperature of less than or equal to - 196°C. In one variation, the container system is frozen by placing it in a cardboard plasma freezing container, freezing it in a chest-type mechanical freezer set at -80°C, and holding it frozen at -65°C. The container system may be frozen for up to 2 to up to 10 years depending on the body fluid.

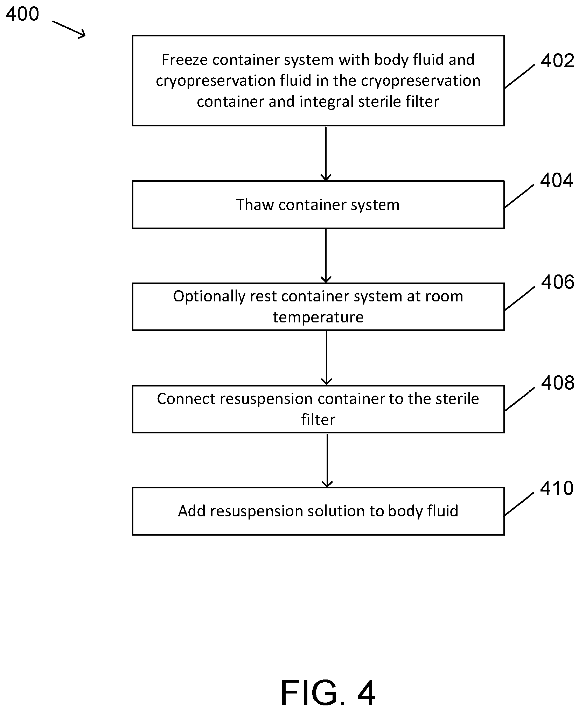

**FIG. 4** provides a method **400** of preparing a frozen body fluid for transfusion into a patient. The method may include freezing the container system at step **402,** thawing the contents of both the cryopreservation container and the resuspension container at step **404,** connecting the resuspension container to the sterile filter at step **408,** and mixing a portion of the resuspension solution into the body fluid at step **410.** Thawing can be with conductive or radiant heat with or without simultaneous mixing. Methods of thawing include, but are not limited to water baths or microwave heating. In an example, frozen CPP are prepared for transfusion by thawing in a 30-37°C water bath and resuspending in a resuspension solution. In a variation, the resuspension solution is sterile 0.9% NaCl for infusion or plasma. The resuspension solution is added at a validated time period following thawing, which may be immediately or after an extended rest of the system. The method may optionally include a rest period of up to 30 minutes at room temperature at step **406** after thawing and before adding the resuspension solution. The need for the rest period may be based on the selection of the resuspension solution.

After the contents of the containers are combined, the connection tubing is sealed with a radio frequency sealer, heat sealer, or clamp. The final product may be held under appropriate conditions and/or transported until the time of transfusion that will not be limited due to a breach of the closed system. It may be limited based on other biological and physiochemical features of the final product such as temperature, cell concentration in the bag, the final resuspension solution, gas permeability of the container and others.

In a variation, the thawed, resuspended body fluid has a shelf life of more than 4 hours. In a variation, the resuspended body fluid has a shelf life of at least 4 hours. In a variation, the resuspended body fluid has a shelf life of at least 12 hours. In a variation, the resuspended body fluid has a shelf life of at least 24 hours. In a variation, the resuspended body fluid has a shelf life of at least 2 days. In a variation, the resuspended body fluid has a shelf life of at least 5 days. In a variation, the resuspended body fluid has a shelf life of at least 2 weeks. In a variation, the resuspended body fluid has a shelf life of at least 1 month. In a variation, the resuspended body fluid has a shelf life of at least 2 months. In a variation, the resuspended body fluid has a shelf life of at least 3 months. In a variation, expiry of the resuspended body fluid may be a function of the resuspension solution type, volume of the resuspended body fluid, the content of the body fluid, etc.

A processes and elements have not been described in order to avoid unnecessarily obscuring the present invention. Accordingly, the above description should not be taken as limiting the scope of the invention.

Those skilled in the art will appreciate that the presently disclosed variations teach by way of example and not by limitation. Therefore, the matter contained in the above description or shown in the accompanying drawings should be interpreted as illustrative and not in a limiting sense. The invention is defined by the appended claims.

## Claims

1. A method of resuspending a cryopreserved body fluid comprising:
thawing a frozen closed and sterile container system (100) comprising:
a cryopreservation container (102) comprising a body fluid in a cryopreservation liquid;
a resuspension container (104) comprising a resuspension solution;
a connection tube (110) sterilely connecting the cryopreservation container (102) and the resuspension container (104); and
a single shut-off element (114) actively associated with the connection tube,
opening the shut-off element (114); and
adding at least a portion of the resuspension solution to the cryopreservation container (102) to create a resuspended body fluid product,
wherein when the container system (100) is frozen, the cryopreservation container (102) and the resuspension container (104) are suitable to remain sterilely connected and the shut-off element (114) is configured to be closed such that the contents of the cryopreservation container and the resuspension container are kept separate, and
wherein when the container system (100) is thawed, the cryopreservation container (102) and the resuspension container (104) are suitable to remain sterilely connected and the shut-off element (114) is configured to be open such that the resuspension solution is added to the cryopreservation container (102), creating a resuspended body fluid product.

2. The method of claim 1, wherein the bodily fluid product is selected from platelets and blood.

3. The method of claim 1 or claim 2, wherein the bodily fluid product is dimethyl sulfoxide (DMSO) cryopreserved platelets (CPP).

4. The method of any one of claims 1 to 3, wherein the connection tube is DMSO compatible.

5. The method of any one of claims 1 to 4, wherein the connection tube and shut-off element are configured to withstand being clamped and frozen at -80° C.

6. The method of any one of claims 1 to 5, wherein the resuspension solution is a transfusable solution.

7. The method of claim 6, wherein the transfusable solution is selected from sterile NaCl, human plasma, and a platelet storage solution.

8. The method of any one of claims 1 to 7, wherein the cryopreservation container is an ethyl vinyl acetate (EVA) freezing bag.

9. The method of claim 1, wherein the resuspended body fluid product remains shelf stable for more than 4 hours.

10. The method of claim 1, wherein the resuspended body fluid product remains shelf stable for up to at least 14 days.

11. The method of claim 1, further comprising transporting the resuspended body fluid product to a site for transfusion.

## Patentansprüche

1. Verfahren zum Resuspendieren eines kryokonservierten Körperfluids, umfassend:
Auftauen eines gefrorenen geschlossenen und sterilen Behältersystems (100) umfassend:
einen Kryokonservierungsbehälter (102), der ein Körperfluid in einer Kryokonservierungsflüssigkeit umfasst;
einen Resuspensionsbehälter (104), der eine Resuspensionslösung umfasst;
ein Verbindungsrohr (110), das den Kryokonservierungsbehälter (102) und den Resuspensionsbehälter (104) steril verbindet; und
ein einziges Absperrelement (114), das aktiv mit dem Verbindungsrohr verbunden ist,
Öffnen des Absperrelements (114); und
Zugeben wenigstens eines Teils der Resuspensionslösung zu dem Kryokonservierungsbehälter (102), um ein resuspendiertes Körperfluidprodukt zu erzeugen,
wobei, wenn das Behältersystem (100) gefroren ist, der Kryokonservierungsbehälter (102) und der Resuspensionsbehälter (104) dazu geeignet sind, steril verbunden zu bleiben, und das Absperrelement (114) dazu ausgelegt ist, so verschlossen zu werden, dass der Inhalt des Kryokonservierungsbehälters und des Resuspensionsbehälters getrennt gehalten werden, und
wobei, wenn das Behältersystem (100) aufgetaut ist, der Kryokonservierungsbehälter (102) und der Resuspensionsbehälter (104) geeignet sind, steril verbunden zu bleiben, und das Absperrelement (114) dazu ausgelegt ist, offen zu sein, sodass die Resuspensionslösung zu dem Kryokonservierungsbehälter (102) zugegeben wird, um ein resuspendiertes Körperfluidprodukt zu erzeugen.

2. Verfahren nach Anspruch 1, wobei das Körperfluidprodukt ausgewählt ist aus Thrombozyten und Blut.

3. Verfahren nach Anspruch 1 oder Anspruch 2, wobei es sich bei dem Körperfluidprodukt um Dimethylsulfoxid(DMSO)-kryokonservierte Thrombozyten (CPP) handelt.

4. Verfahren nach einem der Ansprüche 1 bis 3, wobei das Verbindungsrohr DMSO-kompatibel ist.

5. Verfahren nach einem der Ansprüche 1 bis 4, wobei das Verbindungsrohr und das Absperrelement dazu ausgelegt sind, Klemmen und Einfrieren bei -80° C standzuhalten

6. Verfahren nach einem der Ansprüche 1 bis 5, wobei die Resuspensionslösung eine transfundierbare Lösung ist.

7. Verfahren nach Anspruch 6, wobei die transfundierbare Lösung ausgewählt ist aus sterilem NaCl, menschlichem Plasma und einer Thrombozytenspeicherlösung.

8. Verfahren nach einem der Ansprüche 1 bis 7, wobei der Kryokonservierungsbehälter ein Ethylvinylacetat(EVA)-Gefrierbeutel ist.

9. Verfahren nach Anspruch 1, wobei das resuspendierte Körperfluidprodukt länger als 4 Stunden lagerstabil bleibt.

10. Verfahren nach Anspruch 1, wobei das resuspendierte Körperfluidprodukt bis zu wenigstens 14 Tage lagerstabil bleibt.

11. Verfahren nach Anspruch 1, ferner umfassend Transportieren des resuspendierten Körperfluidprodukts zu einer Stelle zur Transfusion.

## Revendications

1. Procédé de remise en suspension d'un fluide corporel cryoconservé comprenant :
décongélation d'un système de récipient fermé et stérile congelé (100) comprenant :
un récipient (102) de cryoconservation comprenant un fluide corporel dans un liquide de cryoconservation ;
un récipient de remise en suspension (104) comprenant une solution de remise en suspension ;
un tube de connexion (110) reliant stérilement le récipient de cryoconservation (102) et le récipient de remise en suspension (104) ; et
un unique élément d'obturation (114) activement associé au tube de connexion,
ouverture de l'élément d'obturation (114) ; et
ajout d'au moins une partie de la solution de remise en suspension dans le récipient de cryoconservation (102) pour créer un produit de fluide corporel remis en suspension,
dans lequel lorsque le système de récipient (100) est congelé, le récipient de cryoconservation (102) et le récipient de remise en suspension (104) sont adaptés pour rester connectés stérilement et l'élément d'obturation (114) est configuré pour être fermé de sorte que les contenus du récipient de cryoconservation et du récipient de remise en suspension soient maintenus séparés, et
dans lequel lorsque le système de récipient (100) est décongelé, le récipient de cryoconservation (102) et le récipient de remise en suspension (104) sont adaptés pour rester connectés stérilement et l'élément d'obturation (114) est configuré pour être ouvert de sorte que la solution de remise en suspension soit ajoutée au récipient de cryoconservation (102), créant ainsi un produit de fluide corporel remis en suspension.

2. Procédé selon la revendication 1, dans lequel le produit de fluide corporel est choisi parmi les plaquettes et le sang.

3. Procédé selon la revendication 1 ou la revendication 2, dans lequel le produit de fluide corporel est des plaquettes cryoconservées (CPP) dans du diméthylsulfoxyde (DMSO).

4. Procédé selon l'une quelconque des revendications 1 à 3, dans lequel le tube de connexion est compatible avec du DMSO.

5. Procédé selon l'une quelconque des revendications 1 à 4, dans lequel le tube de connexion et l'élément d'arrêt sont configurés pour résister à un serrage et à une congélation à -80 °C.

6. Procédé selon l'une quelconque des revendications 1 à 5, dans lequel la solution de remise en suspension est une solution transférable.

7. Procédé selon la revendication 6, dans lequel la solution transférable est choisie parmi du NaCl stérile, du plasma humain et une solution de stockage de plaquettes.

8. Procédé selon l'une quelconque des revendications 1 à 7, dans lequel le récipient de cryoconservation est un sac de congélation d'éthyle acétate de vinyle (EVA).

9. Procédé selon la revendication 1, dans lequel le produit de fluide corporel remis en suspension reste stable au stockage pendant plus de 4 heures.

10. Procédé selon la revendication 1, dans lequel le produit de fluide corporel remis en suspension reste stable au stockage pendant au moins 14 jours.

11. Procédé selon la revendication 1, comprenant en outre le transport du produit de fluide corporel remis en suspension vers un site pour une transfusion.
